# EUROPEAN PATENT SPECIFICATION

(11) **EP 1 898 951 B1**
(45) Date of publication and mention of the grant of the patent: **27.02.2013**
(21) Application number: 06767753.4
(22) Date of filing: 26.06.2006
(51) Int. Cl.: A61K 45/00, A61P 9/12

(54) **PHARMACEUTICAL PREPARATION CONTAINING AN ANGIOTENSIN II RECEPTOR ANTAGONIST AND A CALCIUM CHANNEL BLOCKER**
PHARMAZEUTISCHES PRÄPARAT MIT EINEM ANGIOTENSIN-II-REZEPTOR-ANTAGONISTEN UND EINEM CALCIUMKANALBLOCKER
PRÉPARATION PHARMACEUTIQUE CONTENANT UN ANTAGONISTE DU RÉCEPTEUR DE L'ANGIOTENSINE II ET UN BLOQUEUR DU CANAL CALCIQUE

(30) Priority: 27.06.2005 JP 2005187251
(43) Date of publication of application: 19.03.2008
(73) Proprietor: Daiichi Sankyo Company, Limited, Chuo-ku Tokyo 103-8426 (JP)
(72) Inventor: HAMAURA, Takeshi, Hiratsuka-shi,Kanagawa 254-0014 (JP); KANNO, Mitsuru, Hiratsuka-shi, Kanagawa 254-0014 (JP)
(74) Representative: Wallace, Sheila Jane
(86) International application number: PCT/JP2006/313175
(87) International publication number: WO 2007/001066

(56) References cited:
- EP-A- 1 604 664
- WO-A-03/035039

## Description

### [TECHNICAL FIELD]

The present invention relates to a pharmaceutical preparation comprising an angiotensin II receptor antagonist and a calcium channel blocker.

### [BACKGROUND ART]

Currently, calcium channel blockers and angiotensin II receptor antagonists are widely used clinically as medicaments for the treatment and prophylaxis of hypertension. Since calcium channel blockers exert natriuretic action in addition to vasodilatory action, they are effective against hypertension caused by fluid retention (renin-independent). On the other hand, angiotensin II receptor antagonists are particularly effective against renin-dependent hypertension, and have excellent organ protective effects. Thus, it is expected that the combined use of a calcium channel blocker and an angiotensin II receptor antagonist should allow stable and effective antihypertensive therapy regardless of the cause of the hypertension.

A number of combination drugs comprising an angiotensin II receptor antagonist and a calcium channel blocker have been proposed in the prior art, such as Patent Documents 1 to 4 below. However, there has been no disclosure in the prior art of combination drugs comprising an angiotensin II receptor antagonist and a calcium channel blocker that additionally comprise a hydrophilic polymer, acidic substance or fluidizing agent to afford improved dissolution properties.
[Patent Document 1] International Publication WO 92/10097
[Patent Document 2] International Publication WO 92/20342
[Patent Document 3] International Publication WO 00/02543
[Patent Document 4] International Publication WO 2004/067003

### [DISCLOSURE OF THE INVENTION]

The object of the present invention is to provide a pharmaceutical preparation with improved dissolution properties comprising the angiotensin II receptor antagonist olmesartan or a pharmacologically acceptable salt or ester thereof, a calcium channel blocker and at least one substance selected from a hydrophilic polymer, an acidic substance and a fluidizing agent.

As a result of conducting extensive research to solve the aforementioned problems, the present inventors found that a pharmaceutical preparation with improved dissolution properties is obtained by incorporation of at least one substance selected from a hydrophilic polymer, an acidic substance that is one or both of tartaric acid and ascorbic acid, and a fluidizing agent in a pharmaceutical preparation comprising olmesartan or a pharmacologically acceptable salt or ester thereof and a calcium channel blocker, thereby leading to completion of the present invention.

The present invention provides a pharmaceutical preparation comprising an angiotensin II receptor antagonist, a calcium channel blocker and at least one substance selected from a hydrophilic polymer, an acidic substance that is one or both of tartaric acid and ascorbic acid, and from 5 to 85% by weight of a fluidizing agent (particularly a preparation for the prophylaxis or treatment of hypertension), the use of an angiotensin II receptor antagonist and a calcium channel blocker to manufacture the aforementioned pharmaceutical preparation (particularly a preparation for the prophylaxis or treatment of hypertension), and a method for preventing or treating a disease (particularly hypertension) in which the aforementioned pharmaceutical preparation comprising pharmacologically effective amounts of an angiotensin II receptor antagonist and a calcium channel blocker is administered to warm-blooded animals (particularly humans), wherein the angiotensin II receptor antagonist is olmesartan or a pharmacologically acceptable salt or ester thereof.

Specifically, the present invention provides:
(1) a pharmaceutical preparation comprising an angiotensin II receptor antagonist, a calcium channel blocker and at least one substance that improves the dissolution properties of said pharmaceutical preparation selected from a hydrophilic polymer, an acidic substance and from 5 to 85% by weight of a fluidizing agent, wherein the angiotensin II receptor antagonist is olmesartan or a pharmacologically acceptable salt or ester thereof and wherein the acidic substance is either or both of tartaric acid and ascorbic acid.
(2) the pharmaceutical preparation according to (1) comprising:
   (A) the angiotensin II receptor antagonist;
   (B) the calcium channel blocker; and
   (C) the hydrophilic polymer,
(3) the pharmaceutical preparation according to (1) comprising:
   (A) the angiotensin II receptor antagonist;
   (B) the calcium channel blocker; and
   (C) the acidic substance,
(4) the pharmaceutical preparation according to (1) comprising:
   (A) the angiotensin II receptor antagonist;
   (B) the calcium channel blocker; and
   (C) the fluidizing agent,
(5) the pharmaceutical preparation according to (1) to (4) wherein the angiotensin II receptor antagonist is olmesartan medoxomil,
(6) the pharmaceutical preparation according to (1) to (5) wherein the calcium channel blocker is nifedipine, nimodipine, nilvadipine, manidipine, barnidipine, nitrendipine, benidipine, nicardipine, lercanidipine, amlodipine, nisoldipine, efonidipine, cilnidipine, azelnidipine, felodipine, aranidipine or pranidipine or a pharmacologically acceptable salt thereof,
(7) the pharmaceutical preparation according to (1) to (5) wherein the calcium channel blocker is manidipine, barnidipine, benidipine, nicardipine, lercanidipine, amlodipine, efonidipine or azelnidipine or a pharmacologically acceptable salt thereof,
(8) the pharmaceutical preparation according to (1) to (5) wherein the calcium channel blocker is amlodipine or a pharmacologically acceptable salt thereof,
(9) the pharmaceutical preparation according to (1) to (5) wherein the calcium channel blocker is amlodipine besylate,
(10) the pharmaceutical preparation according to (1), (2) or (5) to (9) wherein the hydrophilic polymer is at least one compound selected from cellulose derivatives and synthetic polymers,
(11) the pharmaceutical preparation according to (1), (2) or (5) to (9) wherein the hydrophilic polymer is at least one compound selected from hydroxypropyl methyl cellulose, methyl cellulose, hydroxypropyl cellulose, sodium carboxymethyl cellulose, macrogol, HA Sankyo, polyvinylpyrrolidone and polyvinyl alcohol,
(12) the pharmaceutical composition according to (1), (2) or (5) to (9) wherein the hydrophilic polymer is at least one compound selected from cellulose derivatives,
(13) the pharmaceutical preparation according to (1), (2) or (5) to (9) wherein the hydrophilic polymer is at least one compound selected from hydroxypropyl methyl cellulose, methyl cellulose, hydroxypropyl cellulose and sodium carboxymethyl cellulose,
(14) the pharmaceutical preparation according to (1), (2) or (5) to (9) wherein the hydrophilic polymer is either or both of methyl cellulose and hydroxypropyl cellulose,
(15) the pharmaceutical preparation according to (1), (2) or (5) to (9) wherein the hydrophilic polymer is macrogol,
(16) the pharmaceutical preparation according to (1), (4) or (5) to (9) wherein the fluidizing agent is at least one compound selected from calcium silicate, light anhydrous silicic acid, anhydrous calcium hydrogenphosphate, synthetic hydrotalcite and magnesium metasilicate aluminate,
(17) the pharmaceutical preparation according to (1) to (16) wherein the pharmaceutical preparation is a single dosage form,
(18) the pharmaceutical preparation according to (17) wherein the single dosage form is a solid dosage form,
(19) the pharmaceutical preparation according to (18) wherein the solid dosage form is selected from powders, grains, granules, capsules and tablets, and
(20) the pharmaceutical preparation according to (19) wherein the solid dosage form is a tablet.

In addition, a pharmaceutical preparation obtained by arbitrarily combining (1) to (20) above is also preferable, examples of which are indicated below.
(21) the pharmaceutical preparation according to (1) or (2) wherein the angiotensin II receptor antagonist is olmesartan medoxomil,
   the calcium channel blocker is manidipine, barnidipine, benidipine, nicardipine, lercanidipine, amlodipine, efonidipine or azelnidipine or a pharmacologically acceptable salt thereof, arid
   the hydrophilic polymer is at least one compound selected from hydroxypropyl methyl cellulose, methyl cellulose, hydroxypropyl cellulose, sodium carboxymethyl cellulose, macrogol, HA Sankyo, polyvinylpyrrolidone and polyvinyl alcohol,
(22) the pharmaceutical preparation according to (1) or (2) wherein the angiotensin II receptor antagonist is olmesartan medoxomil,
   the calcium channel blocker is amlodipine or a pharmacologically acceptable salt thereof, and
   the hydrophilic polymer is at least one compound selected from hydroxypropyl methyl cellulose, methyl cellulose, hydroxypropyl cellulose, sodium carboxymethyl cellulose, macrogol, HA Sankyo, polyvinylpyrrolidone and polyvinyl alcohol,
(23) the pharmaceutical preparation according to (1) or (2) wherein the angiotensin II receptor antagonist is olmesartan medoxomil,
   the calcium channel blocker is amlodipine or a pharmacologically acceptable salt thereof, and
   the hydrophilic polymer is at least one compound selected from hydroxypropyl methyl cellulose, methyl cellulose, hydroxypropyl cellulose and sodium carboxymethyl cellulose,
(24) the pharmaceutical preparation according to (1) or (2) wherein the angiotensin II receptor antagonist is olmesartan medoxomil,
   the calcium channel blocker is amlodipine or a pharmacologically acceptable salt thereof, and
   the hydrophilic polymer is either or both of methyl cellulose and hydroxypropyl cellulose,
(25) the pharmaceutical preparation according to (1) or (3) wherein the angiotensin II receptor antagonist is olmesartan medoxomil,
   the calcium channel blocker is amlodipine or a pharmacologically acceptable salt thereof, and
   the acidic substance is either or both of tartaric acid and ascorbic acid,
(26) the pharmaceutical preparation according to (1) or (4) wherein the angiotensin II receptor antagonist is olmesartan medoxomil,
   the calcium channel blocker is amlodipine or a pharmacologically acceptable salt thereof, and
   the fluidizing agent is at least one compound selected from calcium silicate, light anhydrous silicic acid, anhydrous calcium hydrogenphosphate, synthetic hydrotalcite and magnesium metasilicate aluminate, and
(27) the pharmaceutical composition according to (21) to (26) wherein the calcium channel blocker is amlodipine besylate.

According to the present invention, a pharmaceutical preparation, that contains olmesartan or a pharmacologically acceptable salt or ester thereof and a calcium channel blocker, and which further comprises at least one substance selected from a hydrophilic polymer, an acidic substance that is one or both of tartaric acid and ascorbic acid, and from 5 to 85% by weight of a fluidizing agent, is provided which has improved dissolution properties.

### [BEST MODE FOR CARRYING OUT THE INVENTION]

The pharmaceutical preparation of the present invention contains an angiotensin II receptor antagonist and a calcium channel blocker as its active ingredients.

Since various medicaments have been proposed as an "angiotensin II receptor antagonist", which is one of the active ingredients in a solid dosage form of the present invention, and many are actually used clinically, a person of ordinary skill in the art can select suitable medicaments that demonstrate the desired effect as an angiotensin II receptor antagonist for use in the present invention. Of these, olmesartan medoxomil is preferably used. Olmesartan medoxomil can easily be produced according to the methods disclosed in the art, suitable examples including the methods disclosed in Japanese Patent No. 2082519 (corresponding to US Patent No. 5,616,599).

Since various medicaments have been proposed as a "calcium channel blocker", which is one of the active ingredients in a solid dosage form of the present invention, and many are actually used clinically, a person of ordinary skill in the art can select suitable medicaments that demonstrate the desired effect as a calcium channel blocker for use in the present invention. Suitable, non-limiting examples of calcium channel blockers for use in the present invention include nifedipine, nimodipine, nilvadipine, manidipine (preferably manidipine hydrochloride), barnidipine (preferably bamidipine hydrochloride), nitrendipine, benidipine (preferably benidipine hydrochloride), nicardipine (preferably nicardipine hydrochloride), lercanidipine (preferably lercanidipine hydrochloride), amlodipine (preferably amlodipine besylate), nisoldipine, efonidipine (preferably efonidipine hydrochloride), cilnidipine, azelnidipine, felodipine, aranidipine and pranidipine. Of these amlodipine besylate is preferably used. Amlodipine and its salts including amlodipine besylate can be easily produced according to the methods disclosed in the art, suitable examples including the methods disclosed in Japanese Patent No. 1401088 (corresponding to US Patent No. 4,572,909).

The pharmacologically acceptable salts of angiotensin II receptor antagonists and a calcium channel blockers described above are not specifically restricted and these salts can be selected by a person of ordinary skill in the art. Suitable pharmacologically acceptable salts include, for example, an alkaline metal salt such as a sodium salt, potassium salt or lithium salt; an alkaline earth metal salt such as a calcium salt or magnesium salt; a metal salt such as an aluminium salt, iron salt, zinc salt, copper salt, nickel salt or cobalt salt; an amine salt such as an ammonium salt, t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt or tris(hydroxymethyl)aminomethane salt; a hydrohalogenic acid salt such as a hydrofluoride, hydrochloride, hydrobromide or hydroiodide; a nitrate; a perchlorate; a sulfate; a phosphate; a C₁-C₄ alkanesulfonic acid salt, which may be optionally substituted with a halogen atom(s) such as a methanesulfonate, trifluoromethanesulfonate or ethanesulfonate; a C₆-C₁₀ arylsulfonic acid salt, which may be optionally substituted with a C₁-C₄ alkyl group(s), such as a benzenesulfonate or p-toluenesulfonate; a C₁-C₆ aliphatic acid salt such as an acetate, malate, fumarate, succinate, citrate, tartrate, oxalate or maleate; or an amino acid salt such as a glycine salt, lysine salt, arginine salt, ornithine salt, glutamic acid salt or aspartic acid salt.

The pharmacologically acceptable esters of the angiotensin II receptor antagonists described above are not particularly restricted, and can be selected by a person of ordinary skill in the art. In the case of said esters, it is preferable that such esters can be cleaved by a biological process such as hydrolysis *in vivo.* The group constituting the esters (the group shown as R when the esters thereof are expressed as - COOR) can be, for example, a C₁-C₄ alkoxy C₁-C₄ alkyl group such as methoxyethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, propoxymethyl, isopropoxymethyl, butoxymethyl or t-butoxymethyl; a C₁-C₄ alkoxylated C₁-C₄ alkoxy C₁-C₄ alkyl group such as 2-methoxyethoxymethyl; a C₆-C₁₀ aryloxy C₁-C₄ alkyl group such as phenoxymethyl; a halogenated C₁-C₄ alkoxy C₁-C₄ alkyl group such as 2,2,2-trichloroethoxymethyl or bis(2-chloroethoxy)methyl; a C₁-C₄ alkoxycarbonyl C₁-C₄ alkyl group such as methoxycarbonylmethyl; a cyano C₁-C₄ alkyl group such as cyanomethyl or 2-cyanoethyl; a C₁-C₄ alkylthiomethyl group such as methylthiomethyl or ethylthiomethyl; a C₆-C₁₀ arylthiomethyl group such as phenylthiomethyl or naphthylthiomethyl; a C₁-C₄ alkylsulfonyl C₁-C₄ lower alkyl group, which may be optionally substituted with a halogen atom(s) such as 2-methanesulfonylethyl or 2-trifluoromethanesulfonylethyl; a C₆-C₁₀ arylsulfonyl C₁-C₄ alkyl group such as 2-benzenesulfonylethyl or 2-toluenesulfonylethyl; a C₁-C₇ aliphatic acyloxy C₁-C₄ alkyl group such as formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl or 1-pivaloyloxyhexyl; a C₅-C₆ cycloalkylcarbonyloxy C₁-C₄ alkyl group such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl 1-cyclohexylcarbonyloxypropyl, 1-cyclopentylcarbonyloxybutyl or 1-cyclohexylcarbonyloxybutyl; a C₆-C₁₀ arylcarbonyloxy C₁-C₄ alkyl group such as benzoyloxymethyl; a C₁-C₆ alkoxycarbonyloxy C₁-C₄ alkyl group such as methoxycarbonyloxymethyl, 1-(methoxycarbonyloxy)ethyl, 1-(methoxycarbonyloxy)propyl, 1-(methoxycarbonyloxy)butyl, 1-(methoxycarbonyloxy)pentyl, 1-(methoxycarbonyloxy)hexyl, ethoxycarbonyloxymethyl, 1-(ethoxycarbonyloxy)ethyl, 1-(ethoxycarbonyloxy)propyl, 1-(ethoxycarbonyloxy)butyl, 1-(ethoxycarbonyloxy)pentyl,1-(ethoxycarbonyloxy)hexyl, propoxycarbonyloxymethyl, 1-(propoxycarbonyloxy)ethyl, 1-(propoxycarbonyloxy)propyl, 1-(propoxycarbonyloxy)butyl, isopropoxycarbonyloxymethyl 1-(isopropoxycarbonyloxy)ethyl, 1-(isopropoxycarbonyloxy)butyl, butoxycarbonyloxymethyl, 1-(butoxycarbonyloxy)ethyl, 1-(butoxycarbonyloxy)propyl, 1-(butoxycarbonyloxy)butyl, isobutoxycarbonyloxymethyl, 1-(isobutoxycarbonyloxy)ethyl, 1-(isobutoxycarbonyloxy)propyl, 1-(isobutoxycarbonyloxy)butyl, t-butoxycarbonyloxymethyl, 1-(t-butoxycarbonyloxy)ethyl, pentyloxycarbonyloxymethyl, 1-(pentyloxycarbonyloxy)ethyl, 1-(pentyloxycarbonyloxy)propyl, hexyloxycarbonyloxymethy, 1-(hexyloxycarbonyloxy)ethyl or 1-(hexyloxycarbonyloxy)propyl; a C₅-C₆ cycloalkyloxycarbonyloxy C₁-C₄ alkyl group such as cyclopentyloxycarbonyloxymethyl, 1-(cyclopentyloxycarbonyloxy)ethyl, 1-(cyclopentyloxycarbonyloxy)propyl, 1-(cyclopentyloxycarbonyloxy)butyl, cyclohexyloxycarbonyloxymethyl, 1-(cyclohexyloxycarbonyloxy)ethyl, 1-(cyclohexyloxycarbonyloxy)propyl or 1-(cyclohexyloxycarbonyloxy)butyl; a [5-(C₁-C₄ alkyl)-2-oxo-1,3-dioxolen-4-yl]methyl group such as (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl or (5-butyl-2-oxo-1,3-dioxolen-4-yl)methy; a [5-(phenyl, which may be optionally substituted with a C₁-C₄ alkyl, C₁-C₄ alkoxy or halogen atom(s))-2-oxo-1,3-dioxolen-4-yl]methyl group such as (5-phenyl-2-oxo-1,3-dioxolen-4-yl)methyl, [5-(4-methylphenyl)-2-oxo-1,3-dioxolen4-yl]methyl, [5-(4-methoxyphenyl)-2-oxo-1,3-dioxolen-4-yl]methyl, [5-(4-fluorophenyl)-2-oxo-1,3-dioxolen-4-yl]methyl or [5-(4-chlorophenyl)-2-oxo-1,3-dioxolen-4-yl]methyl; or a phthalidyl group, which may be optionally substituted with a C₁-C₄ alkyl or C₁-C₄ alkoxy group(s), such as phthalidyl, dimethylphthalidyl or dimethoxyphthalidyl.

The "hydrophilic polymers" in the pharmaceutical preparations of the present invention are polymers that have an affinity for water. Preferred "hydrophilic polymers" for use in the present invention are ones which are water-soluble. Suitable, non-limiting examples of hydrophilic polymers for use in the present invention include cellulose derivatives such as hydroxypropyl methyl cellulose, methyl cellulose, hydroxypropyl cellulose and sodium carboxymethyl cellulose; synthetic polymers such as polyvinylpyrrolidone, aminoalkyl methacrylate copolymer, carboxyvinyl polymer, polyvinyl alcohol and macrogol (i.e. polyethylene glycol); HA Sankyo (a pre-mixed coating agent comprising a mixture of 16-26% by weight of polyvinyl acetal diethyl aminoacetate, 50-75% by weight of hydroxypropylmethyl cellulose 2910, 12-17% by weight of stearic acid and 1.5-2.3% by weight of fumaric acid), gum Arabic, agar, gelatin and sodium alginate. Of these, hydroxypropyl methyl cellulose, methyl cellulose, hydroxypropyl cellulose, sodium carboxymethyl cellulose, macrogol, HA Sankyo, polyvinylpyrrolidone and polyvinyl alcohol are preferred, hydroxypropyl methyl cellulose, methyl cellulose, hydroxypropyl cellulose, macrogol and sodium carboxymethyl cellulose are more preferred, and methyl cellulose is most preferred. In the present invention, these hydrophilic polymers can be used alone or two or more kinds can be used in combination. Where at least one hydrophilic polymer is present in the pharmaceutical preparation of the present invention, said hydrophilic polymer (or polymers) is preferably present in an amount of from 1 to 90% by weight of the total weight of the pharmaceutical preparation, and more preferably from 5 to 85% by weight. The one or more hydrophilic polymers may be uniformly distributed throughout the entire pharmaceutical preparation, or they may be contained in only a part of said pharmaceutical preparation. If one or more film coating layers are present in the pharmaceutical preparation, the one or more hydrophilic polymers may be contained in said film coating layers.

As "acidic substances" for use in the pharmaceutical preparations of the present invention, the inorganic acids tartaric acid and ascorbic acid are used. In the present invention, these acidic substances can be used alone or both can be used in combination. Where at least one acidic substance is present in the pharmaceutical preparation of the present invention, said acidic substance (or acidic substances) is preferably present in an amount of from 1 to 90% by weight of the total weight of the pharmaceutical preparation, and more preferably from 5 to 85% by weight.

The "fluidizing agents" in the pharmaceutical preparations of the present invention are flow-modifying agents that increase the fluidity of the other ingredients present in said preparations. Suitable, non-limiting examples of "fluidizing agents" for use in the pharmaceutical preparations of the present invention include hydrous silicon dioxide, light anhydrous silicic acid, microcrystalline cellulose, synthetic aluminum silicate, alumina, magnesium hydroxide, stearic acid, calcium stearate, magnesium stearate, tribasic calcium phosphate, talc, concentrated glycerin, Perfiller 101, anhydrous ethanol, calcium silicate, anhydrous calcium hydrogenphosphate, synthetic hydrotalcite and magnesium metasilicate aluminate. Of these, calcium silicate, light anhydrous silicic acid, anhydrous calcium hydrogenphosphate, synthetic hydrotalcite and magnesium metasilicate aluminate are preferred. In the present invention, these can be used alone or two or more kinds can be used in combination. Where at least one fluidizing agent is present in the pharmaceutical preparation of the present invention, said fluidizing agent (or fluidizing agents) is present in an amount of from 5 to 85% by weight of the total weight of the pharmaceutical preparation.

The pharmaceutical preparation of the present invention can where desired additionally contain at least one further additive such as a suitable pharmacologically acceptable excipient, lubricant, binder, disintegrant, emulsifier, stabilizer, corrective or diluent.

Suitable "excipients" include organic excipients including sugar derivatives such as lactose, sucrose, glucose, mannitol or sorbitol; starch derivatives such as corn starch, potato starch, α-starch or dextrin; cellulose derivatives such as microcrystalline cellulose; gum Arabic; dextran; and pullulan, and inorganic excipients including silicate derivatives such as light anhydrous silicic acid, synthetic aluminum silicate, calcium silicate or magnesium metasilicate aluminate; phosphates such as dibasic calcium hydrogenphosphate; carbonates such as calcium carbonate; and sulfates such as calcium sulfate.

Suitable "lubricants" include stearic acid; stearic acid metal salts such as calcium stearate or magnesium stearate; talc; colloidal silica; waxes such as beeswax or spermaceti; boric acid; adipic acid; sulfates such as sodium sulfate; glycol; fumaric acid; sodium benzoate; D,L-leucine; lauryl sulfates such as sodium lauryl sulfate or magnesium lauryl sulfate; silicates such as silicic anhydride or silicate hydrate; and the aforementioned starch derivatives.

Suitable "binders" include hydroxypropyl cellulose, hydroxypropyl methyl cellulose, polyvinylpyrrolidone, macrogol and compounds similar to the aforementioned excipients.

Suitable "disintegrants" include cellulose derivatives such as low-substituted hydroxypropyl cellulose, carboxymethyl cellulose, calcium carboxymethyl cellulose or internally crosslinked sodium carboxymethyl cellulose; cross-linked polyvinylpyrrolidone; and chemically modified starches/celluloses such as carboxymethyl starch or sodium carboxymethyl starch.

Suitable "emulsifiers" include colloidal clays such as bentonite or bee gum; metal hydroxides such as magnesium hydroxide or aluminum hydroxide; anionic surfactants such as sodium lauryl sulfate or calcium stearate; cationic surfactants such as benzalkonium chloride; and nonionic surfactants such as polyoxyethylene alkyl ether, polyoxyethylene sorbitan fatty acid ester or sucrose fatty acid ester.

Suitable "stabilizers" include para-hydroxybenzoic acid esters such as methyl paraben or propyl paraben; alcohols such as chlorobutanol, benzyl alcohol or phenyl ethyl alcohol; benzalkonium chloride; phenols such as phenol or cresol; thimerosal; dehydroacetic acid; and sorbic acid.

Suitable "correctives" include sweeteners such as sodium saccharin or aspartame; sour flavourings such as citric acid, malic acid or tartaric acid; and fragrances such as menthol, lemon or orange fragrance.

Suitable "diluents" include lactose, mannitol, glucose, sucrose, calcium sulfate, calcium phosphate, hydroxypropyl cellulose, microcrystalline cellulose, water, ethanol, polyethylene glycol, propylene glycol, glycerol, starch, polyvinylpyrrolidone, magnesium metasilicate aluminate, and mixtures thereof.

The pharmaceutical preparation of the present invention may be a preparation in which the angiotensin II receptor antagonist and the calcium channel blocker are provided in separate pharmaceutical dosage forms (e.g. solid dosage forms), one or both of which further comprise one or more substances selected from hydrophilic polymers, acidic substances and, fluidizing agents, i.e. the pharmaceutical preparation is provided as a "kit of parts" wherein the separate pharmaceutical dosage forms are either administered to the patient together or at a suitable time interval such that they are able to act together in the desired manner. Alternatively, the pharmaceutical preparation of the present invention comprises the angiotensin II receptor antagonist, the calcium channel blocker and the one or more substances selected from hydrophilic polymers, acidic substances and fluidizing agents are provided together in a single dosage form, Preferably, the pharmaceutical preparation of the present invention is a single dosage form.

The pharmaceutical preparation of the present invention is preferably a solid dosage form, either as separate solid dosage forms in a "kit of parts" or, preferably, as a single solid dosage form. Suitable solid dosage forms will be well known to the person skilled in the art, and non-limiting examples of the solid dosage form of the present invention include tablets (including sublingual tablets and tablets that disintegrate in the mouth), capsules (including soft capsules and microcapsules), granules, grains, powders, pills and lozenges. Of these, powders, grains, granules, capsules and tablets are preferred, and tablets are most preferred.

A dosage form of the present invention may be produced using any commonly used method well known to persons skilled in the art of pharmaceutical formulation technology and there are no particular limitations thereon. Examples of suitable methods include those disclosed in publications such as Powder Technology and Pharmaceutical Processes [D. Chulia et al., Elsevier Science Pub. Co. (December 1, 1993)].

A tablet of the present invention can be obtained, for example, by granulating, drying and sizing a principal agent with a vehicle, binder and so forth using a suitable method well known in the art, adding a lubricant and so forth to the resulting mixture followed by mixing and forming into a tablet. Granulation can be carried out by any suitable method well known in the art such as wet granulation, dry granulation or heated granulation. Suitable, non-limiting examples include these granulation techniques carried out using a high-speed agitation granulator, a fluidized granulation dryer, an extrusion granulator or a roller compactor. In addition, procedures such as drying and sizing may be carried out as necessary following granulation. A mixture of the principal agent, vehicle, binder, lubricant and so forth can also be directly formed into tablets. Furthermore, a tablet of the present invention may also be provided with at least one layer of a film coating.

If a film coating is desired, any film coating apparatus of a type well known in the art can be used, and as film coating bases, suitable examples include sugar coating bases, hydrophilic film coating bases, enteric film coating bases and sustained release film coating bases.

Suitable examples of sugar coating bases include saccharose, and these can be used in combination with one or more additives such as talc, precipitated calcium carbonate, calcium phosphate, calcium sulfate, gelatin, gum Arabic, polyvinylpyrrolidone and pullulan.

Suitable examples of hydrophilic film coating bases include cellulose derivatives such as hydroxypropyl cellulose, hydroxypropyl methyl cellulose, hydroxyethyl cellulose, methyl hydroxyethyl cellulose and sodium carboxymethyl cellulose; synthetic polymers such as polyvinyl acetal diethyl aminoacetate, aminoalkyl methacrylate copolymer, polyvinylpyrrolidone and macrogol; and polysaccharides such as pullulan.

Suitable examples of enteric film coating bases include cellulose derivatives such as hydroxypropyl methyl cellulose, phthalate hydroxypropyl methyl cellulose acetate succinate, carboxymethyl ethyl cellulose and cellulose acetate phthalate; acrylic acid derivatives such as methacrylic acid copolymer L, methacrylic acid copolymer LD and methacrylic acid copolymer S; and natural substances such as shellac.

Suitable examples of sustained release film coating bases include cellulose derivatives such as ethyl cellulose; and acrylic acid derivatives such as aminoalkyl methacrylate copolymer RS, ethyl acrylate-methyl methacrylate copolymer emulsion.

A mixture of two or more different coating bases such as those above may also be used in a suitable ratio. In addition, the coating films may also contain suitable pharmacologically acceptable additives such as plasticizers, excipients, lubricants, opacifying agents, colorants or antiseptics as necessary.

The doses and the dosing ratios of the angiotensin II receptor antagonist and calcium channel blocker, which are the active ingredients in the pharmaceutical preparation of the present invention, can be changed depending on various factors such as the activity of each of the active ingredients and the symptoms, age and body weight of the patient. Although the dosage varies depending on symptoms, age and the like, the dose of each class of active ingredient in the case of oral administration is typically from 0.001 mg/kg (preferably 0.01 mg/kg) per day as a lower limit to 10 mg/kg (preferably 1 mg/kg) per day as an upper limit for a human adult, and the dosage can be administered from one to six times per day depending on the symptoms of the patients.

In addition, the dosing ratio of the angiotensin II receptor antagonist and calcium channel blocker, which are the active ingredients in the pharmaceutical preparation of the present invention, can also be changed over a wide range. For example, the dosing ratio by weight of angiotensin II receptor antagonist and calcium channel blocker can typically be within a range of 1:1000 to 1000:1, preferably within a range of 1:100 to 100:1, and more preferably within a range of 1:10 to 10:1.

The pharmaceutical preparation of the present invention is effective for the prophylaxis or treatment of, for example, hypertension or diseases caused by hypertension [more specifically, hypertension, heart disease (angina pectoris, myocardial infarction, arrhythmia, cardiac insufficiency or hypercardia), kidney disease (diabetic nephropathy, glomerular nephritis or nephrosclerosis), or cerebrovascular disease (cerebral infarction or cerebral hemorrhage)] and the like.

### [EXAMPLES]

The present invention will be described in more detail by way of the following examples, but the scope of the present invention is not limited thereto.

### Example 1

Olmesartan medoxomil, amlodine besylate, lactose, low substituted hydroxypropyl cellulose, microcrystalline cellulose, methyl cellulose and magnesium stearate were each weighed out in the relative amounts given in column 1 of Table 1 below, and they were then mixed for 2 minutes in an agate mortar. The resulting mixture was formed into tablets using a hydraulic single-action tablet press with a stamp having a 7 mm diameter flat surface at a tablet weight of 140 mg and pressing pressure of 10 kN. The dissolution properties of the resulting tablets were tested according to the procedure shown in Test Example 1 below and the results are shown in column 1 of the following Table 2.

### Example 2

A similar procedure to Example 1 was carried out using the relative amounts given in column 2 of Table 1, the methyl cellulose of Example 1 being replaced with hydroxypropyl cellulose. The dissolution properties of the resulting tablets were tested according to the procedure shown in Test Example 1 below and the results are shown in column 2 of the following Table 2.

### Example 3

A similar procedure to Example 1 was carried out using the relative amounts given in column 3 of Table 1, the methyl cellulose of Example 1 being replaced with hydroxypropyl methyl cellulose. The dissolution properties of the resulting tablets were tested according to the procedure shown in Test Example 1 below and the results are shown in column 3 of the following Table 2.

### Reference Example 1

A similar procedure to Example 1 was carried out using the relative amounts given in column 4 of Table 1, in which the methyl cellulose used in Example 1 is excluded and additional lactose is used in its place. The dissolution properties of the resulting tablets were tested according to the procedure shown in Test Example 1 below and the results are shown in column 4 of the following Table 2.

### Test Example 1

Testing for the rate of dissolution of the tablets prepared in Examples 1 to 3 and Reference Example 1 was carried out in accordance with Method 2 of the Dissolution Test (Paddle Method) described in the 14th Revised Edition of the Japanese Pharmacopoeia at 50 revolutions per minute and using 900 mL of Japanese Pharmacopoeia Solution 2 (JP-2) for the test solution. The test solution was sampled at 30 minutes and 60 minutes after the start of testing followed by measurement of the dissolution rate and dissolved amount of olmesartan medoxomil by absorption spectrometry (dissolution tester: Toyama Sangyo; spectrophotometer: Shimadzu). Testing was carried out on two tablets and their average value is indicated in each case.

**(Table 1)**

| No. | 1 | 2 | 3 | 4 |
|---|---|---|---|---|
| Example/Reference Example | Example 1 | Example 2 | Example 3 | Reference Example 1 |
| Olmesartan medoxomil | 10 | 10 | 10 | 10 |
| Amlodipine besylate | 13.86 | 13.86 | 13.86 | 13.86 |
| Lactose | 71.14 | 71.14 | 71.14 | 85.14 |
| Low substituted hydroxypropyl cellulose | 20 | 20 | 20 | 20 |
| Microcrystalline cellulose | 10 | 10 | 10 | 10 |
| Methyl cellulose | 14 | | | |
| Hydroxypropyl cellulose | | . 14 | | |
| Hydroxypropyl methyl cellulose | | | 14 | |
| Magnesium stearate | 1 | 1 | 1 | 1 |
| Total (mg/tablet) | 140 | 140 | 140 | 140 |

**(Table 2)**

| No. | 1 | 2 | 3 | 4 |
|---|---|---|---|---|
| Example/Reference Example | Example 1 | Example 2 | Example 3 | Reference Example 1 |
| Dissolution rate after 30 minutes (%) (based on a value of 100% for Reference Example 1) | 68.9 | 66.5 | 61.5 | 50.3 |
| | (137.0%) | (132.2%) | (122.2%) | (100.0%) |
| Amount dissolved after 30 minutes (µg/mL) | 7.7 | 7.4 | 6.8 | 5.6 |
| Dissolution rate after 60 minutes (%) (based on a value of 100% for Reference Example 1) | 77.5 | 76.0 | 70.2 | 56.5 |
| | (137.2%) | (134.5%) | (124.2%) | (100.0%) |
| Amount dissolved after 60 minutes (µg/mL) | 8.6 | 8.4 | 7.8 | 6.3 |

### Example 4

A similar procedure to Example 1 was carried out using the relative amounts given in column 1 of Table 3, the methyl cellulose of Example 1 being replaced with tartaric acid. The dissolution properties of the resulting tablets were tested according to the procedure shown in Test Example 2 below and the results are shown in column 1 of the following Table 4.

### Example 5

A similar procedure to Example 1 was carried out using the relative amounts given in column 2 of Table 3, the methyl cellulose of Example 1 being replaced with ascorbic acid. The dissolution properties of the resulting tablets were tested according to the procedure shown in Test Example 2 below and the results are shown in column 2 of the following Table 4.

### Example 6

A similar procedure to Example 1 was carried out using the relative amounts given in column 3 of Table 3, the methyl cellulose of Example 1 being replaced with calcium silicate. The dissolution properties of the resulting tablets were tested according to the procedure shown in Test Example 2 below and the results are shown in column 3 of the following Table 4.

### Example 7

A similar procedure to Example 1 was carried out using the relative amounts given in column 4 of Table 3, the methyl cellulose of Example 1 being replaced with light anhydrous silicic acid. The dissolution properties of the resulting tablets were tested according to the procedure shown in Test Example 2 below and the results are shown in column 4 of the following Table 4.

### Example 8

A similar procedure to Example 1 was carried out using the relative amounts given in column 5 of Table 3, the methyl cellulose of Example 1 being replaced with anhydrous calcium hydrogenphosphate. The dissolution properties of the resulting tablets were tested according to the procedure shown in Test Example 2 below and the results are shown in column 5 of the following Table 4.

### Example 9

A similar procedure to Example 1 was carried out using the relative amounts given in column 6 of Table 3, the methyl cellulose of Example 1 being replaced with synthetic hydrotalcite. The dissolution properties of the resulting tablets were tested according to the procedure shown in Test Example 2 below and the results are shown in column 6 of the following Table 4.

### Example 10

A similar procedure to Example 1 was carried out using the relative amounts given in column 7 of Table 3, the methyl cellulose of Example 1 being replaced with magnesium metasilicate aluminate. The dissolution properties of the resulting tablets were tested according to the procedure shown in Test Example 2 below and the results are shown in column 7 of the following Table 4.

### Reference Example 2

A similar procedure to Example 1 was carried out using the relative amounts given in column 8 of Table 3, in which the methyl cellulose from Example 1 is excluded and additional lactose is used in its place. The dissolution properties of the resulting tablets were tested according to the procedure shown in Test Example 2 below and the results are shown in column 8 of the following Table 4.

### Test Example 2

Testing for the rate of dissolution of the tablets prepared in Examples 4 to 10 and Reference Example 2 was carried out in accordance with Method 2 of the Dissolution Test (Paddle Method) described in the 14th Revised Edition of the Japanese Pharmacopoeia at 50 revolutions per minute and using 900 mL of Japanese Pharmacopoeia Solution 2 (JP-2) for the test solution. The test solution was sampled at 30 minutes and 60 minutes after the start of testing followed by measurement of the dissolution rate and dissolved amount of olmesartan medoxomil by absorption spectrometry (dissolution tester: Toyama Sangyo; spectrophotometer: Shimadzu). Testing was carried out on two tablets and their average value is indicated.

**(Table 3)**

| No. | 1 | 2 | 3 | 4 | 5 | 6 | 7 | 8 |
|---|---|---|---|---|---|---|---|---|
| Example/Reference Example | Example 4 Tartaric acid | Example 5 Ascorbic acid | Example 6 Calcium silicate | Example 7 Light anhydrous silicic acid | Example 8 Anhydrous dibasic calcium phosphate | Example 9 Synthetic hydrotalcite | Example 10 Magnesium metasilicate aluminate | Reference Example 2 |
| Olmesartan medoxomil | 10 | 10 | 10 | 10 | 10 | 10 | 10 | 10 |
| Amlodipine besylate | 13.86 | 13.86 | 13.86 | 13.86 | 13.86 | 13.86 | 13.86. | 13.86 |
| Lactose | 75.14 | 75.14 | 75.14 | 75.14 | 75.14 | 75.14 | 75.14 | 85.14 |
| Low substituted hydroxypropyl cellulose | 20 | 20 | 20 | 20 | 20 | 20 | 20 | 20 |
| Microcrystalline cellulose | 10 | 10 | 10 | 10 | 10 | 10 | 10 | 10 |
| Various additives | 10 | 10 | 10 | 10 | 10 | 10 | 10 | |
| Magnesium stearate | 1 | 1 | 1 | 1 | 1 | 1 | 1 | 1 |
| Total (mg/tablet) | 140 | 140 | 140 | 140 | 140 | 140 | 140 | 140 |

**(Table 4)**

| No. | 1 | 2 | 3 | 4 | 5 | 6 | 7 | 8 |
|---|---|---|---|---|---|---|---|---|
| Example/Reference Example | Example 4 | Example5 | Example 6 | Example 7 | Example 8 | Example 9 | Example 10 | Reference Example 2 |
| Dissolution rate after 30 minutes (%) (based a value of 100% for Reference Example 1) | 89.5 (177.9%) | 83.1 (165.2%) | 92.5 (183.9%) | 88.7 (176.3%) | 81.7 (162.4%) | 59.7 (118.7%) | 92.7 (184.3%) | 50.3 (100.0%) |
| Amount dissolved after 30 minutes (µg/mL) | 9.9 | 9.2 | 10.3 | 9.9 | 9.1 | 6.6 | 10.3 | 5.6 |
| Dissolution rate after 60 minutes (%) (based on a value of 100% for Reference Example 1) | 9L7 (162.3%) | 87.5 (154.9%) | 95.2 (168.5%) | 92.1 (163.0%) | 89.2 (157.9%) | 69.9 (123.7%) | 95.6 (169.2%) | 56.5 (100.0%) |
| Amount dissolved after 60 minutes (µg/mL) | 10.2 | 9.1 | 10.6 | 10.2 | 9.9 | 7.8 | 10.6 | 6.3 |

As shown in Tables 2 and 4 above, the pharmaceutical preparations of the present invention demonstrate superior dissolution properties for the angiotensin II receptor antagonist (olmesartan medoxomil) contained therein.

### [INDUSTRIAL APPLICABILITY]

According to the present invention a pharmaceutical composition, comprising olmesartan or a pharmacologically acceptable salt or ester thereof and a calcium channel blocker, and further comprising at least one substance selected from hydrophilic polymers, acidic substances that are either or both of tartaric acid and ascorbic acid, and fluidizing agents, is provided which has improved dissolution properties.

## Claims

1. A pharmaceutical preparation comprising an angiotensin II receptor antagonist, a calcium channel blocker and at least one substance that improves the dissolution properties of said pharmaceutical preparation selected from a hydrophilic polymer, an acidic substance and from 5 to 85% by weight of a fluidizing agent, wherein the angiotensin II receptor antagonist is olmesartan or a pharmacologically acceptable salt or ester thereof and wherein the acidic substance is either or both of tartaric acid and ascorbic acid.

2. A pharmaceutical preparation according to claim 1 comprising the angiotensin II receptor antagonist, the calcium channel blocker and the hydrophilic polymer.

3. A pharmaceutical preparation according to claim 1 comprising the angiotensin II receptor antagonist, the calcium channel blocker and the acidic substance.

4. A pharmaceutical preparation according to claim 1 comprising the angiotensin II receptor antagonist, the calcium channel blocker and the fluidizing agent.

5. The pharmaceutical preparation according to any one of claims 1 to 4 wherein the angiotensin II receptor antagonist is olmesartan medoxomil.

6. The pharmaceutical preparation according to any one of claims 1 to 5 wherein the calcium channel blocker is nifedipine, nimodipine, nilvadipine, manidipine, barnidipine, nitrendipine, benidipine, nicardipine, lercanidipine, amlodipine, nisoldipine, efonidipine, cilnidipine, azelnidipine, felodipine, aranidipine or pranidipine or a pharmacologically acceptable salt thereof.

7. The pharmaceutical preparation according to any one of claims 1 to 5 wherein the calcium channel blocker is manidipine, barnidipine, benidipine, nicardipine, lercanidipine, amlodipine, efonidipine or azelnidipine or a pharmacologically acceptable salt thereof.

8. The pharmaceutical preparation according to any one of claims 1 to 5 wherein the calcium channel blocker is amlodipine or a pharmacologically acceptable salt thereof.

9. The pharmaceutical preparation according to any one of claims 1 to 5 wherein the calcium channel blocker is amlodipine besylate.

10. The pharmaceutical preparation according to any one of claims 1, 2 and 5 to 9 wherein the hydrophilic polymer is at least one compound selected from cellulose derivatives and synthetic polymers.

11. The pharmaceutical preparation according to any one of claims 1, 2 and 5 to 9 wherein the hydrophilic polymer is at least one compound selected from hydroxypropyl methyl cellulose, methyl cellulose, hydroxypropyl cellulose, sodium carboxymethyl cellulose, macrogol, HA Sankyo, polyvinylpyrrolidone and polyvinyl alcohol.

12. The pharmaceutical preparation according to any one of claims 1, 2 and 5 to 9 wherein the hydrophilic polymer is at least one compound selected from cellulose derivatives.

13. The pharmaceutical preparation according to any one of claims 1, 2 and 5 to 9 wherein the hydrophilic polymer is at least one compound selected from hydroxypropyl methyl cellulose, methyl cellulose, hydroxypropyl cellulose and sodium carboxymethyl cellulose.

14. The pharmaceutical preparation according to any one of claims 1, 2 and 5 to 9 wherein the hydrophilic polymer is either or both of methyl cellulose and hydroxypropyl cellulose.

15. The pharmaceutical preparation according to any one of claims 1, 2 and 5 to 9 wherein the hydrophilic polymer is macrogol.

16. The pharmaceutical preparation according to any one of claims 1 and 4 to 9 wherein the fluidizing agent is at least one compound selected from calcium silicate, light anhydrous silicic acid, anhydrous calcium hydrogenphosphate, synthetic hydrotalcite and magnesium metasilicate aluminate.

17. The pharmaceutical preparation according to claim 1 or claim 2, wherein the angiotensin II receptor antagonist is olmesartan medoxomil,
the calcium channel blocker is manidipine, barnidipine, benidipine, nicardipine, lercanidipine, amlodipine, efonidipine or azelnidipine or a pharmacologically acceptable salt thereof, and
the hydrophilic polymer is at least one compound selected from hydroxypropyl methyl cellulose, methyl cellulose, hydroxypropyl cellulose, sodium carboxymethyl cellulose, macrogol, HA Sankyo, polyvinylpyrrolidone and polyvinyl alcohol.

18. The pharmaceutical preparation according to claim 1 or claim 2 wherein the angiotensin II receptor antagonist is olmesartan medoxomil,
the calcium channel blocker is amlodipine or a pharmacologically acceptable salt thereof, and
the hydrophilic polymer is at least one compound selected from hydroxypropyl methyl cellulose, methyl cellulose, hydroxypropyl cellulose, sodium carboxymethyl cellulose, macrogol, HA Sankyo, polyvinylpyrrolidone and polyvinyl alcohol.

19. The pharmaceutical preparation according to claim 1 or claim 2 wherein the angiotensin II receptor antagonist is olmesartan medoxomil,
the calcium channel blocker is amlodipine or a pharmacologically acceptable salt thereof, and
the hydrophilic polymer is at least one compound selected from hydroxypropyl methyl cellulose, methyl cellulose, hydroxypropyl cellulose and sodium carboxymethyl cellulose.

20. The pharmaceutical preparation according to claim 1 or claim 2 wherein the angiotensin II receptor antagonist is olmesartan medoxomil,
the calcium channel blocker is amlodipine or a pharmacologically acceptable salt thereof, and
the hydrophilic polymer is either or both of methyl cellulose and hydroxypropyl cellulose.

21. The pharmaceutical preparation according to claim 1 or claim 3 wherein the angiotensin II receptor antagonist is olmesartan medoxomil,
the calcium channel blocker is amlodipine or a pharmacologically acceptable salt thereof, and
the acidic substance is either or both of tartaric acid and ascorbic acid.

22. The pharmaceutical preparation according to claim 1 or claim 4 wherein the angiotensin II receptor antagonist is olmesartan medoxomil,
the calcium channel blocker is amlodipine or a pharmacologically acceptable salt thereof, and
the fluidizing agent is at least one compound selected from calcium silicate, light anhydrous silicic acid, anhydrous calcium hydrogenphosphate, synthetic hydrotalcite and magnesium metasilicate aluminate.

23. The pharmaceutical preparation according to any one of claims 17 to 22 wherein the calcium channel blocker is amlodipine besylate.

24. The pharmaceutical preparation according to any one of claims 1 to 23 wherein the pharmaceutical preparation is a single dosage form.

25. The pharmaceutical preparation according to claim 24 wherein the single dosage form is a solid dosage form.

26. The pharmaceutical preparation according to claim 25 wherein the solid dosage form is selected from powders, grains, granules, capsules and tablets.

27. The pharmaceutical preparation according to claim 26 wherein the solid dosage form is a tablet.

28. A pharmaceutical preparation according to any one of claims 1 to 27 for the prophylaxis or treatment of hypertension.

29. The use of an angiotensin II receptor antagonist and a calcium channel blocker in the manufacture of a medicament for the prophylaxis or treatment of hypertension, wherein said medicament is a pharmaceutical preparation according to any one of claims 1 to 27.

## Patentansprüche

1. Pharmazeutisches Präparat umfassend einen Angiotensin-II-Rezeptor-Antagonisten, einen Calciumkanalblocker und mindestens eine Substanz, die die Löseeigenschaften des pharmazeutischen Präparats verbessert, ausgewählt unter einem hydrophilen Polymer, einer sauren Substanz und 5 bis 85 Gew.-% eines Fluidisierungsmittels, wobei der Angiotensin-II-Rezeptor-Antagonist Olmesartan oder ein pharmakologisch akzeptables Salz oder ein pharmakologisch akzeptabler Ester davon ist und wobei die saure Substanz entweder eines oder beides von Weinsäure und Ascorbinsäure ist.

2. Pharmazeutisches Präparat nach Anspruch 1, umfassend den Angiotensin-II-Rezeptor-Antagonisten, den Calciumkanalblocker und das hydrophile Polymer.

3. Pharmazeutisches Präparat nach Anspruch 1, umfassend den Angiotensin-II-Rezeptor-Antagonisten, den Calciumkanalblocker und die saure Substanz.

4. Pharmazeutisches Präparat nach Anspruch 1, umfassend den Angiotensin-II-Rezeptor-Antagonisten, den Calciumkanalblocker und das Fluidisierungsmittel.

5. Pharmazeutisches Präparat nach einem der Ansprüche 1 bis 4, wobei der Angiotensin-II-Rezeptor-Antagonist Olmesartanmedoxomil ist.

6. Pharmazeutisches Präparat nach einem der Ansprüche 1 bis 5, wobei der Calciumkanalblocker Nifedipin, Nimodipin, Nilvadipin, Manidipin, Barnidipin, Nitrendipin, Benidipin, Nicardipin, Lercanidipin, Amlodipin, Nisoldipin, Efonidipin, Cilnidipin, Azelnidipin, Felodipin, Aranidipin oder Pranidipin oder ein pharmakologisch akzeptables Salz davon ist.

7. Pharmazeutisches Präparat nach einem der Ansprüche 1 bis 5, wobei der Calciumkanalblocker Manidipin, Barnidipin, Benidipin, Nicardipin, Lercanidipin, Amlodipin, Efonidipin oder Azelnidipin oder ein pharmakologisch akzeptables Salz davon ist.

8. Pharmazeutisches Präparat nach einem der Ansprüche 1 bis 5, wobei der Calciumkanalblocker Amlodipin oder ein pharmakologisch akzeptables Salz davon ist.

9. Pharmazeutisches Präparat nach einem der Ansprüche 1 bis 5, wobei der Calciumkanalblocker Amlodipinbesylat ist.

10. Pharmazeutisches Präparat nach einem der Ansprüche 1, 2 und 5 bis 9, wobei das hydrophile Polymer mindestens eine Verbindung ist ausgewählt unter Cellulosederivaten und synthetischen Polymeren.

11. Pharmazeutisches Präparat nach einem der Ansprüche 1, 2 und 5 bis 9,wobei das hydrophile Polymer mindestens eine Verbindung ist ausgewählt unter Hydroxypropylmethylcellulose, Methylcellulose, Hydroxypropylcellulose, Natriumcarboxymethylcellulose, Macrogol, HA Sankyo, Polyvinylpyrrolidon und Polyvinylalkohol.

12. Pharmazeutisches Präparat nach einem der Ansprüche 1, 2 und 5 bis 9, wobei das hydrophile Polymer mindestens eine Verbindung ist ausgewählt unter Cellulosederivaten.

13. Pharmazeutisches Präparat nach einem der Ansprüche 1, 2 und 5 bis 9, wobei das hydrophile Polymer mindestens eine Verbindung ist ausgewählt unter Hydroxypropylmethylcellulose, Methylcellulose, Hydroxypropylcellulose und Natriumcarboxymethylcellulose.

14. Pharmazeutisches Präparat nach einem der Ansprüche 1, 2 und 5 bis 9, wobei das hydrophile Polymer entweder eines oder beides von Methylcellulose und Hydroxypropylcellulose ist.

15. Pharmazeutisches Präparat nach einem der Ansprüche 1, 2 und 5 bis 9, wobei das hydrophile Polymer Macrogol ist.

16. Pharmazeutisches Präparat nach einem der Ansprüche 1 und 4 bis 9, wobei das Fluidisierungsmittel mindestens eine Verbindung ist ausgewählt unter Calciumsilicat, leichter wasserfreier Kieselsäure, wasserfreiem Calciumhydrogenphosphat, synthetischem Hydrotalcit und Magnesiummetasilicataluminat.

17. Pharmazeutisches Präparat nach Anspruch 1 oder Anspruch 2, wobei der Angiotensin-II-Rezeptor-Antagonist Olmesartanmedoxomil ist,
der Calciumkanalblocker Manidipin, Bamidipin, Benidipin, Nicardipin, Lercanidipin, Amlodipin, Efonidipin oder Azelnidipin oder ein pharmakologisch akzeptables Salz davon ist und
das hydrophile Polymer mindestens eine Verbindung ist ausgewählt unter Hydroxypropylmethylcellulose, Methylcellulose, Hydroxypropylcellulose, Natriumcarboxymethylcellulose, Macrogol, HA Sankyo, Polyvinylpyrrolidon und Polyvinylalkohol.

18. Pharmazeutisches Präparat nach Anspruch 1 oder Anspruch 2, wobei der Angiotensin-II-Rezeptor-Antagonist Olmesartanmedoxomil ist,
der Calciumkanalblocker Amlodipin oder ein pharmakologisch akzeptables Salz davon ist und
das hydrophile Polymer mindestens eine Verbindung ist ausgewählt unter Hydroxypropylmethylcellulose, Methylcellulose, Hydroxypropylcellulose, Natriumcarboxymethylcellulose, Macrogol, HA Sankyo, Polyvinylpyrrolidon und Polyvinylalkohol.

19. Pharmazeutisches Präparat nach Anspruch 1 oder Anspruch 2, wobei der Angiotensin-II-Rezeptor-Antagonist Olmesartanmedoxomil ist,
der Calciumkanalblocker Amlodipin oder ein pharmakologisch akzeptables Salz davon ist und
das hydrophile Polymer mindestens eine Verbindung ist ausgewählt unter Hydroxypropylmethylcellulose, Methylcellulose, Hydroxypropylcellulose und Natriumcarboxymethylcellulose.

20. Pharmazeutisches Präparat nach Anspruch 1 oder Anspruch 2, wobei der Angiotensin-II-Rezeptor-Antagonist Olmesartanmedoxomil ist,
der Calciumkanalblocker Amlodipin oder ein pharmakologisch akzeptables Salz davon ist und
das hydrophile Polymer entweder eines oder beides von Methylcellulose und Hydroxypropylmethylcellulose ist.

21. Pharmazeutisches Präparat nach Anspruch 1 oder Anspruch 3, wobei der Angiotensin-II-Rezeptor-Antagonist Olmesartanmedoxomil ist,
der Calciumkanalblocker Amlodipin oder ein pharmakologisch akzeptables Salz davon ist und
die saure Substanz entweder eines oder beides von Weinsäure und Ascorbinsäure ist.

22. Pharmazeutisches Präparat nach Anspruch 1 oder Anspruch 4, wobei der Angiotensin-II-Rezeptor-Antagonist Olmesartanmedoxomil ist,
der Calciumkanalblocker Amlodipin oder ein pharmakologisch akzeptables Salz davon ist und
das Fluidisierungsmittel mindestens eine Verbindung ist ausgewählt unter Calciumsilicat, leichter wasserfreier Kieselsäure, wasserfreiem Calciumhydrogenphosphat, synthetischem Hydrotalcit und Magnesiummetasilicataluminat.

23. Pharmazeutisches Präparat nach einem der Ansprüche 17 bis 22, wobei der Calciumkanalblocker Amlodipinbesylat ist.

24. Pharmazeutisches Präparat nach einem der Ansprüche 1 bis 23, wobei das pharmazeutische Präparat eine Einzeldosisform ist.

25. Pharmazeutisches Präparat nach Anspruch 24, wobei die Einzeldosisform eine feste Dosisform ist.

26. Pharmazeutisches Präparat nach Anspruch 25, wobei die feste Dosisform unter Pulvern, Körnern, Granulat, Kapseln und Tabletten ausgewählt wird.

27. Pharmazeutisches Präparat nach Anspruch 26, wobei die feste Dosisform eine Tablette ist.

28. Pharmazeutisches Präparat nach einem der Ansprüche 1 bis 27 für die Prophylaxe oder Behandlung von Bluthochdruck.

29. Verwendung eines Angiotensin-II-Rezeptor-Antagonisten und eines Calciumkanalblockers bei der Herstellung eines Medikaments für die Prophylaxe oder Behandlung von Bluthochdruck, wobei das Medikament ein pharmazeutisch akzeptables Präparat nach einem der Ansprüche 1 bis 27 ist.

## Revendications

1. Préparation pharmaceutique comprenant un antagoniste du récepteur de l'angiotensine II, un agent bloquant du canal calcique et au moins une substance qui améliore les propriétés de dissolution de ladite préparation pharmaceutique sélectionnés parmi un polymère hydrophile, une substance acide et de 5 à 85 % en poids d'un agent de fluidification, dans laquelle l'antagoniste du récepteur de l'angiotensine II est l'olmesartan ou son sel ou ester pharmacologiquement acceptable et dans laquelle la substance acide est l'un ou l'autre ou les deux parmi l'acide tartarique et l'acide ascorbique.

2. Préparation pharmaceutique selon la revendication 1, comprenant l'antagoniste du récepteur de l'angiotensine II, l'agent bloquant du canal calcique et le polymère hydrophile.

3. Préparation pharmaceutique selon la revendication 1, comprenant l'antagoniste du récepteur de l'angiotensine II, l'agent bloquant du canal calcique et la substance acide.

4. Préparation pharmaceutique selon la revendication 1, comprenant l'antagoniste du récepteur de l'angiotensine II, l'agent bloquant du canal calcique et l'agent de fluidification.

5. Préparation pharmaceutique selon l'une quelconque des revendications 1 à 4, dans laquelle l'antagoniste du récepteur de l'angiotensine II est l'olmesartan médoxomil.

6. Préparation pharmaceutique selon l'une quelconque des revendications 1 à 5, dans laquelle l'agent bloquant du canal calcique est la nifédipine, la nimodipine, la nilvadipine, la manidipine, la barnidipine, la nitrendipine, la benidipine, la nicardipine, la lercanidipine, l'amlodipine, la nisoldipine, l'efonidipine, la cilnidipine, l'azelnidipine, la felodipine, l'aranidipine ou la pranidipine ou son sel pharmacologiquement acceptable.

7. Préparation pharmaceutique selon l'une quelconque des revendications 1 à 5, dans laquelle l'agent bloquant du canal calcique est la manidipine, la barnidipine, la benidipine, la nicardipine, la lercanidipine, l'amlodipine, l'efonidipine ou l'azelnidipine ou son sel pharmacologiquement acceptable.

8. Préparation pharmaceutique selon l'une quelconque des revendications 1 à 5, dans laquelle l'agent bloquant du canal calcique est l'amlodipine ou son sel pharmacologiquement acceptable.

9. Préparation pharmaceutique selon l'une quelconque des revendications 1 à 5, dans laquelle l'agent bloquant du canal calcique est le besylate d'amlodipine.

10. Préparation pharmaceutique selon l'une quelconque des revendications 1, 2 et 5 à 9, dans laquelle le polymère hydrophile est au moins un composé sélectionné parmi les dérivés de cellulose et les polymères synthétiques.

11. Préparation pharmaceutique selon l'une quelconque des revendications 1, 2 et 5 à 9, dans laquelle le polymère hydrophile est au moins un composé sélectionné parmi l'hydroxypropyl méthyl cellulose, la méthyl cellulose, l'hydroxypropyl cellulose, la carboxyméthyl cellulose sodique, le macrogol, le HA Sankyo, la polyvinylpyrrolidone et le poly(alcool de vinyle).

12. Préparation pharmaceutique selon l'une quelconque des revendications 1, 2 et 5 à 9, dans laquelle le polymère hydrophile est au moins un composé sélectionné parmi les dérivés de cellulose.

13. Préparation pharmaceutique selon l'une quelconque des revendications 1, 2 et 5 à 9, dans laquelle le polymère hydrophile est au moins un composé sélectionné parmi l'hydroxypropyl méthyl cellulose, la méthyl cellulose, l'hydroxypropyl cellulose et la carboxyméthyl cellulose sodique.

14. Préparation pharmaceutique selon l'une quelconque des revendications 1, 2 et 5 à 9, dans laquelle le polymère hydrophile est l'un ou l'autre ou les deux parmi la méthyl cellulose et l'hydroxypropyl cellulose.

15. Préparation pharmaceutique selon l'une quelconque des revendications 1, 2 et 5 à 9, dans laquelle le polymère hydrophile est le macrogol.

16. Préparation pharmaceutique selon l'une quelconque des revendications 1 et 4 à 9, dans laquelle l'agent de fluidification est au moins un composé sélectionné parmi le silicate de calcium, l'acide silicique légèrement anhydre, l'hydrogénophosphate de calcium anhydre, l'hydrotalcite synthétique et le métasilicate aluminate de magnésium.

17. Préparation pharmaceutique selon la revendication 1 ou la revendication 2, dans laquelle l'antagoniste du récepteur de l'angiotensine II est l'olmesartan médoxomil,
l'agent bloquant du canal calcique est la manidipine, la barnidipine, la benidipine, la nicardipine, la lercanidipine, l'amlodipine, l'efonidipine ou l'azelnidipine ou son sel pharmacologiquement acceptable, et
le polymère hydrophile est au moins un composé sélectionné parmi l'hydroxypropyl méthyl cellulose, la méthyl cellulose, l'hydroxypropyl cellulose, la carboxyméthyl cellulose sodique, le macrogol, le HA Sankyo, la polyvinylpyrrolidone et le poly(alcool de vinyle).

18. Préparation pharmaceutique selon la revendication 1 ou la revendication 2, dans laquelle l'antagoniste du récepteur de l'angiotensine II est l'olmesartan médoxomil,
l'agent bloquant du canal calcique est l'amlodipine ou son sel pharmacologiquement acceptable, et
le polymère hydrophile est au moins un composé sélectionné parmi l'hydroxypropyl méthyl cellulose, la méthyl cellulose, l'hydroxypropyl cellulose, la carboxyméthyl cellulose sodique, le macrogol, le HA Sankyo, la polyvinylpyrrolidone et le poly(alcool de vinyle).

19. Préparation pharmaceutique selon la revendication 1 ou la revendication 2, dans laquelle l'antagoniste du récepteur de l'angiotensine II est l'olmesartan médoxomil,
l'agent bloquant du canal calcique est l'amlodipine ou son sel pharmacologiquement acceptable, et
le polymère hydrophile est au moins un composé sélectionné parmi l'hydroxypropyl méthyl cellulose, la méthyl cellulose, l'hydroxypropyl cellulose et la carboxyméthyl cellulose sodique.

20. Préparation pharmaceutique selon la revendication 1 ou la revendication 2, dans laquelle l'antagoniste du récepteur de l'angiotensine II est l'olmesartan médoxomil,
l'agent bloquant du canal calcique est l'amlodipine ou son sel pharmacologiquement acceptable, et
le polymère hydrophile est l'un ou l'autre ou les deux parmi la méthyl cellulose et l'hydroxypropyl cellulose.

21. Préparation pharmaceutique selon la revendication 1 ou la revendication 3, dans laquelle l'antagoniste du récepteur de l'angiotensine II est l'olmesartan médoxomil,
l'agent bloquant du canal calcique est l'amlodipine ou son sel pharmacologiquement acceptable, et
la substance acide est l'un ou l'autre ou les deux parmi l'acide tartarique et l'acide ascorbique.

22. Préparation pharmaceutique selon la revendication 1 ou la revendication 4, dans laquelle l'antagoniste du récepteur de l'angiotensine II est l'olmesartan médoxomil,
l'agent bloquant du canal calcique est l'amlodipine ou son sel pharmacologiquement acceptable, et
l'agent de fluidification est au moins un composé sélectionné parmi le silicate de calcium, l'acide silicique légèrement anhydre, l'hydrogénophosphate de calcium anhydre, l'hydrotalcite synthétique et le métasilicate aluminate de magnésium.

23. Préparation pharmaceutique selon l'une quelconque des revendications 17 à 22, dans laquelle l'agent bloquant du canal calcique est le besylate d'amlodipine.

24. Préparation pharmaceutique selon l'une quelconque des revendications 1 à 23, dans laquelle la préparation pharmaceutique est une forme dosifiée unique.

25. Préparation pharmaceutique selon la revendication 24, dans laquelle la forme dosifiée unique est une forme dosifiée solide.

26. Préparation pharmaceutique selon la revendication 25, dans laquelle la forme dosifiée solide est sélectionnée parmi les poudres, les grains, les granules, les capsules et les comprimés.

27. Préparation pharmaceutique selon la revendication 26, dans laquelle la forme dosifiée solide est un comprimé.

28. Préparation pharmaceutique selon l'une quelconque des revendications 1 à 27, pour la prophylaxie ou le traitement de l'hypertension.

29. Utilisation d'un antagoniste du récepteur de l'angiotensine II et d'un agent bloquant du canal calcique dans la fabrication d'un médicament pour la prophylaxie ou le traitement de l'hypertension, dans laquelle ledit médicament est une préparation pharmaceutique selon l'une quelconque des revendications 1 à 27.
